# EUROPÄISCHE PATENTANMELDUNG

(11) **EP 1 726 273 A1**
(43) Veröffentlichungstag der Anmeldung: **29.11.2006**
(21) Anmeldenummer: 05011259.8
(22) Anmeldetag: 24.05.2005
(51) Int. Cl.: A61F 2/36

(54) **Hüftgelenkprothese**

(71) Anmelder: Zimmer GmbH, 8404 Winterthur (CH)
(72) Erfinder: Grappiolo, Guido, 17024 Finale Ligure (IT); Willi, Roland, 8413 Neftenbach (CH)
(74) Vertreter: Manitz, Finsterwald & Partner GbR

(57) **Zusammenfassung**

Die Erfindung betrifft eine Hüftgelenkprothese mit einem in den Femurhals einsetzbaren Prothesenshaft (11), der mit seinem proximalen, insbesondere konusförmig ausgebildeten Endbereich (13) mit einem Prothesenkopf (15) koppelbar ist, für den eine in den Hüftknochen einsetzbare Prothesenschale vorgesehen ist, wobei der Schaft an seinem Umfang wirksame Verankerungsmittel (17,19) aufweist, über welche der Schaft in einem durch den Femurhals verlaufenden Durchgang (21) verankerbar ist, in den der Schaft von lateral her einbringbar ist. Die Erfindung betrifft außerdem einen Instrumentensatz zum Einsetzen einer derartigen Hüftgelenkprothese.

## Beschreibung

Die Erfindung betrifft eine Hüftgelenkprothese mit einem in den Femurhals einsetzbaren Prothesenschaft, der mit seinem proximalen, insbesondere konusförmig ausgebildeten Endbereich mit einem Prothesenkopf koppelbar ist, für den eine in den Hüftknochen einsetzbare Prothesenschale vorgesehen ist.

Es sind Hüftgelenkprothesen grundsätzlich bekannt, bei denen sich der Prothesenschaft nicht wie bei schaftfixierten Prothesen in den Femurschaft (Corpus femoris) hinein erstreckt, sondern ohne Verankerung im Femurschaft durch den Femurhals (Collum femoris) verläuft. Derartige Prothesen werden auch als Schenkelhalsprothesen bezeichnet.

Aufgabe der Erfindung ist es, eine Hüftgelenkprothese der eingangs genannten Art zu schaffen, die auf möglichst einfache und sichere Art und Weise am Femur verankert werden kann, wobei insbesondere ausschließlich minimal invasive Operationstechniken zum Einsatz kommen sollen.

Die Lösung dieser Aufgabe erfolgt durch die Merkmale des Anspruchs 1.

Erfindungsgemäß weist der Schaft an seinem Umfang wirksame Verankerungsmittel auf, über welche der Schaft in einem durch den Femurhals verlaufenden Durchgang verankerbar ist, in den der Schaft von lateral her einbringbar ist.

Durch die erfindungsgemäß am Schaftumfang wirksamen Verankerungsmittel kann die Verankerung des Schaftes innerhalb des im Femur ausgebildeten Durchgangs erfolgen. Es ist daher nicht erforderlich, für eine Fixierung des Schaftes an der Außenseite des Femurs zu sorgen, wie es insbesondere bei den bekannten Druckscheibenprothesen der Fall ist. Bei derartigen Prothesen wird der freie Endbereich des Prothesenschaftes mit einer Druckscheibe verbunden, die in Verlängerung des Femurhalses lateral außen auf den Knochen drückt und hierdurch den Prothesenschaft fixiert. Die Erfindung dagegen erleichtert und vereinfacht die Operation, da die Verankerung des Prothesenschaftes innerhalb des im Femur ausgebildeten Durchgangs erfolgt. Insbesondere ist von Vorteil, dass das Einsetzen der erfindungsgemäßen Prothese durch minimal invasive Techniken erfolgen kann.

Bevorzugte Ausführungsformen der Erfindung sind auch in den abhängigen Ansprüchen, der Beschreibung sowie der Zeichnung angegeben.

Vorzugsweise umfassen die Verankerungsmittel ein am Umfang des Schaftes ausgebildetes Gewinde, über welches der Schaft in den Durchgang eingedreht werden kann.

In einer besonders bevorzugten Ausgestaltung ist der Schaft mit einem Zementierabschnitt versehen, über den eine Verankerung des Schaftes im Femur durch Einzementieren erfolgen kann. Hierzu ist der Schaft insbesondere mit wenigstens einer Vertiefung an seinem Umfang zur Aufnahme von Knochenzement versehen. Die Fixierung des Prothesenschaftes im Femur kann hierbei also durch die kombinierte Wirkung eines Gewindes und einer Zementverankerung erfolgen.

Vorzugsweise ist die Vertiefung zur Einbringung des Knochenzements über das distale Ende des Schaftes zugänglich. Hierdurch kann das Einzementieren bzw. das Einbringen des Knochenzementes bei bereits im Durchgang befindlichem Prothesenschaft erfolgen.

Die Vertiefung kann insbesondere eine lang gestreckte Form aufweisen und parallel zur Schaftachse verlaufen.

In einer weiteren bevorzugten Ausgestaltung ist eine Mehrzahl von in Umfangsrichtung verteilt angeordneten Vertiefungen vorgesehen, die jeweils durch einen insbesondere rippen- oder stegartigen Wandabschnitt voneinander getrennt sind. Der mit den Vertiefungen versehene Verankerungsabschnitt hat dabei insbesondere einen kreuz- oder sternförmigen Querschnitt.

Der das Gewinde umfassende Verankerungsabschnitt des Schaftes ist vorzugsweise zwischen dem proximalen Kopplungsabschnitt für den Prothesenkopf einerseits und einem distalen, die Vertiefung umfassenden Verankerungsabschnitt (im Folgenden auch: Zementierabschnitt) andererseits gelegen. Der Zementierabschnitt kann sich bis zum distalen Ende des Schaftes erstrecken. In jedem Fall ist insbesondere vorgesehen, dass der Zementierabschnitt den distalen oder hinteren Bereich des Schaftes bildet, und zwar auch dann, wenn der Zementierabschnitt nicht bis zum distalen Ende des Schaftes reicht.

In einer möglichen konkreten Ausgestaltung ist der Schaft im Bereich des die Vertiefung umfassenden Verankerungsabschnitts mit einem von seinem distalen Ende ausgehenden und in die Vertiefung mündenden Kanal zum Einbringen des Knochenzements versehen. Hierbei gelangt der Knochenzement also in die Vertiefung nicht direkt, sondern über den Kanal. Hierdurch kann der zwischen der Vertiefung und dem freien Schaftende gelegene Bereich des Schaftes frei gestaltet werden.

Vorzugsweise ist für eine Mehrzahl von Vertiefungen ein gemeinsamer Kanal vorgesehen. Bevorzugt ist ein zentral verlaufender Kanal vorgesehen, dessen Mittelachse mit der Schaftachse zusammenfällt.

Insbesondere dann, wenn ein Kanal zum Einbringen des Knochenzements vorgesehen ist, kann der Schaft an seinem distalen Ende mit einem Dichtungsabschnitt versehen sein, der in radialer Richtung über den die Vertiefung umfassenden Verankerungsabschnitt hinausragt. Hierdurch wird ein Austreten von Knochenzement aus dem Durchgang verhindert, wenn zum Einbringen des Knochenzements beispielsweise ein entsprechendes Einbringinstrument auf das hintere Ende des Schaftes aufgesetzt wird, um den Knochenzement über den Kanal in die Vertiefung einzubringen, von wo aus der Zement in das offenporige Knochenmaterial (Spongiosa) gelangen kann, um auf diese Weise im distalen Bereich des Schaftes für dessen sichere Verankerung im Knochen zu sorgen.

Die Aufteilung der für den erfindungsgemäßen Prothesenschaft vorgesehenen Verankerungsmittel in - vereinfacht ausgedrückt - einen proximalen Gewindeabschnitt und einen distalen Zementierabschnitt ist in idealer Weise auf den Femuraufbau abgestimmt. Der Gewindeabschnitt wirkt mit dem harten Material der Kortikalis am Femurhals zusammen, wohingegen der Zementierabschnitt für eine Verankerung im offenporigen Material der Spongiosa sorgt, indem der eingebrachte Knochenzement über die Vertiefung in den Knochen gelangt und sich dort insbesondere "wolkenartig" ausbreitet, so dass nach dem Erhärten des Zements ein optimaler Halt des Schaftes im distalen Bereich gegeben ist.

Der am distalen Ende des Schaftes vorgesehene Dichtungsabschnitt kann als Gewindeabschnitt ausgebildet sein. Hierdurch kann von dem im Durchgang ausgebildeten Innengewinde auch mit dem distalen Ende des Schaftes Gebrauch gemacht werden, wodurch eine stabile Fixierung und insbesondere korrekte Ausrichtung des Schaftes befördert wird.

Der Dichtungsabschnitt kann aus einem bioresorbierbaren Material hergestellt sein.

In einer weiteren bevorzugten Ausgestaltung ist der Schaft mehrteilig ausgebildet. Vorzugsweise ist ein das Gewinde umfassender Schaftteil aus einem anderen Material hergestellt als ein die Vertiefung umfassender Schaftteil.

Die Erfindung betrifft auch einen Instrumentensatz zum Einsetzen einer insbesondere die Merkmale eines der vorhergehenden Ansprüche aufweisenden Hüftgelenkprothese, die einen in den Femurhals einsetzbare Prothesenschaft umfasst, der mit seinem proximalen, insbesondere konusförmig ausgebildeten Endbereich mit einem Prothesenkopf koppelbar ist, für den eine in den Hüftknochen einsetzbare Prothesenschale vorgesehen ist, wobei der Schaft an seinem Umfang wirksame Verankerungsmittel aufweist, über welche der Schaft in einem durch den Femurhals verlaufenden Durchgang verankerbar ist, in den der Schaft von lateral her einbringbar ist, und wobei der Instrumentensatz einen Kernlochbohrer und einen Gewindeschneider zum Herstellen des Durchgangs sowie ein Eindrehinstrument zum Eindrehen des Schaftes in den Durchgang von lateral aufweist.

Vorzugsweise ist zusätzlich ein Einbringinstrument zum Einbringen von Knochenzement vorgesehen, das einen auf das distale Ende des Schaftes aufsetzbaren Einbringabschnitt aufweist. Der Einbringabschnitt kann nach Art einer Tülle ausgebildet sein und dient insbesondere als Adapter zur optimalen Anpassung des Ausströmendes einer herkömmlichen Zementspritze oder -pistole an das distale Ende des Prothesenschaftes.

Der Einbringabschnitt kann derart auf das distale Ende des Schaftes abgestimmt sein, dass im aufgesetzten Zustand ein im Schaft ausgebildeter Kanal gegenüber der Umgebung abgedichtet ist.

Insbesondere kann der Einbringabschnitt zumindest bereichsweise kappen-, hauben- oder glockenförmig ausgebildet sein.

Vorzugsweise handelt es sich bei dem Einbringabschnitt um ein separates, mit einer Zementspritze oder -pistole verbindbares Bauteil.

Die Erfindung kann bei einem Verfahren zum Einsetzen einer Hüftgelenkprothese der vorstehend beschriebenen Art eingesetzt werden, bei dem über einen vorderen Zugang und entsprechenden Schnitt der Femurkopf entfernt, von lateral ein durch den Femurhals verlaufendes Kernloch ausgebildet, in das Kernloch ein Gewinde geschnitten, in den dadurch gebildeten Durchgang der Prothesenschaft eingedreht und über den vorderen Zugang der Prothesenkopf auf den Schaft aufgesetzt wird.

Vorzugsweise wird der Schaft einzementiert. Insbesondere wird der Knochenzement bei bereits im Durchgang befindlichem Schaft eingebracht.

Zum Einzementieren des Schaftes wird vorzugsweise auf dessen distales Ende ein insbesondere tüllenartig ausgebildeter Einbringabschnitt eines Einbringinstruments aufgesetzt.

Die Bearbeitung des Acetabulum (Hüftgelenkspfanne) des Hüftknochens zum Einsetzen der Prothesenschale erfolgt insbesondere über den vorderen Zugang (anterior), über den vorher der Femurkopf entfernt wird.

Alternativ ist es auch möglich, diese Bearbeitung über das zuvor ausgebildete Kernloch bzw. den fertigen Durchgang durchzuführen. Hierbei kann in das Kernloch bzw. den fertigen Durchgang eine Hülse eingeschoben werden, durch welche anschließend Spindeln für die notwendigen Bearbeitungsinstrumente geführt und die eigentlichen Instrumente auf die durchgesteckten Spindeln aufgesteckt werden. Diese Hülse kann in vorteilhafter Weise zur Fixierung des Femur genutzt werden, indem das Femur über einen nach außen vorstehenden Abschnitt der Hülse in einer korrekt relativ zum Hüftknochen ausgerichteten Stellung insbesondere am Operationstisch fixiert wird.

Die Prothesenschale wird insbesondere über den vorderen Zugang, über den der Femurkopf entfernt wird, eingesetzt, und zwar insbesondere nach dem Ausbilden des Kernlochs und vor dem Schneiden des Gewindes.

Ein besonderer Vorteil der erfindungsgemäßen Verankerung des Prothesenschaftes mittels eines im Durchgang ausgebildeten Gewindes besteht darin, dass insbesondere nach dem Aufsetzen des Prothesenkopfes eine Lagekorrektur durch Verdrehen des Schaftes im Durchgang vorgenommen werden kann.

Die Erfindung wird im Folgenden beispielhaft unter Bezugnahme auf die Zeichnung beschrieben. Es zeigen:
- Fig. 1: verschiedene Ansichten eines Prothesenschaftes einer erfindungsgemäßen Hüftgelenkprothese gemäß einer ersten Ausführungsform,
- Fig. 2: einen Gewindeschneider eines erfindungsgemäßen Instrumentensatzes,
- Fig. 3: das Eindrehen des Prothesenschaftes der ersten Ausführungsform mit einem erfindungsgemäßen Eindrehinstrument,
- Fig. 4: das Einbringen von Knochenzement bei einem Prothesenschaft der ersten Ausführungsform mit einem erfindungsgemäßen Einbringinstrument,
- Fig. 5: einen Prothesenschaft der ersten Ausführungsform im eingesetzten Zustand mit aufgesetztem Prothesenkopf,
- Fig. 6: verschiedene Ansichten eines Prothesenschaftes einer erfindungsgemäßen Hüftgelenkprothese gemäß einer zweiten Ausführungsform,
- Fig. 7: das Eindrehen des Prothesenschaftes der zweiten Ausführungsform mit einem anderen erfindungsgemäßen Eindrehinstrument,
- Fig. 8: das Einbringen von Knochenzement bei einem Prothesenschaft der zweiten Ausführungsform mit einem anderen erfindungsgemäßen Einbringinstrument, und
- Fig. 9: einen Prothesenschaft der zweiten Ausführungsform im eingesetzten Zustand mit aufgesetztem Prothesenkopf.

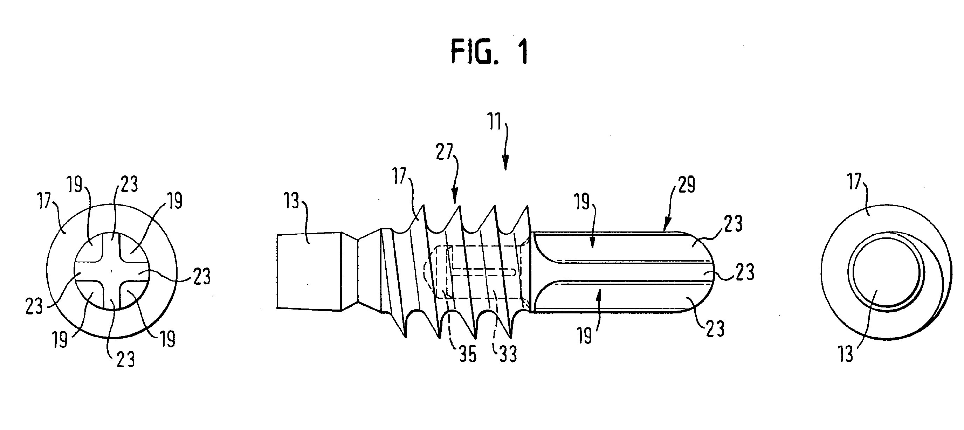

Von der erfindungsgemäßen Hüftgelenkprothese gemäß der ersten Ausführungsform ist in Fig. 1 lediglich der Prothesenschaft 11 dargestellt. Der Schaft 11 ist mehrteilig ausgeführt und umfasst einen distalen Zementierabschnitt 29 und einen proximalen Abschnitt, der am proximalen Ende als Konus 13 ausgebildet ist sowie einen sich nach distal an den Konus 13 anschließenden, mit einem Außengewinde 17 versehenen Abschnitt 27 umfasst.

Der Konus 13 dient zur Kopplung mit einem hier nicht dargestellten, eine entsprechend geformte Aufnahme für den Konus 13 aufweisenden Prothesenkopf. Ferner umfasst die erfindungsgemäße Prothese eine hier ebenfalls nicht dargestellte Gelenkschale zum Einsetzen in den Hüftknochen.

Zur Bildung des Schaftes 11 sind die beiden beschriebenen Schaftteile 27, 29 fest zusammengesteckt. Hierzu ist der Zementierabschnitt 29 mit einem Verbindungszapfen 33 versehen, für den im Gewindeabschnitt 27 eine entsprechende Zapfenaufnahme 35 ausgebildet ist.

Der Zementierabschnitt 29 besitzt einen sternförmigen Querschnitt, der durch vier jeweils paarweise rechtwinklig zueinander stehende, sich in axialer Richtung erstreckende Stege 23 gebildet ist, die in axialer Richtung lang gestreckte, nach distal offene Vertiefungen 19 zur Aufnahme von Knochenzement in Umfangsrichtung begrenzen.

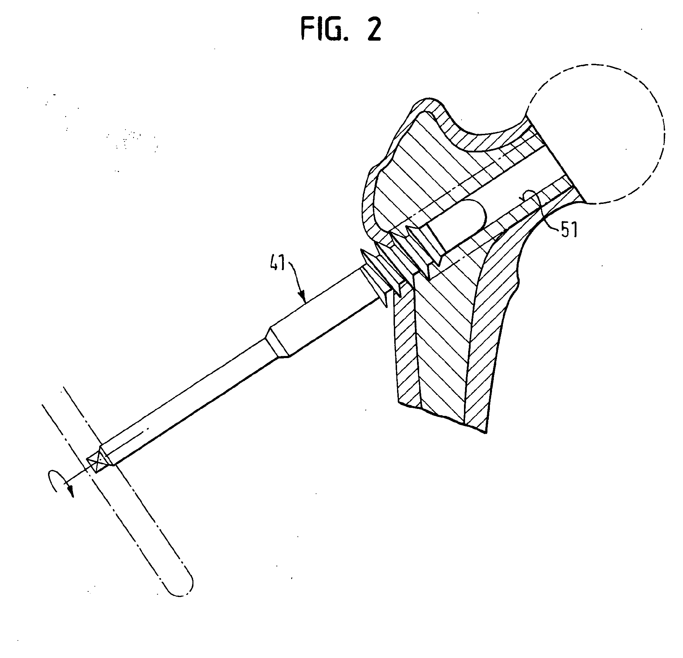

Fig. 2 zeigt, wie am Femur von lateral kommend in Verlängerung der Femurhalsachse mittels eines Gewindeschneiders 41 ein Gewinde in ein zuvor ausgebildetes Kernloch 51 geschnitten wird. Der Femurkopf ist lediglich gestrichelt dargestellt, da er in dieser Phase des Operationsverfahrens, auf das nachstehend näher eingegangen wird, bereits entfernt ist.

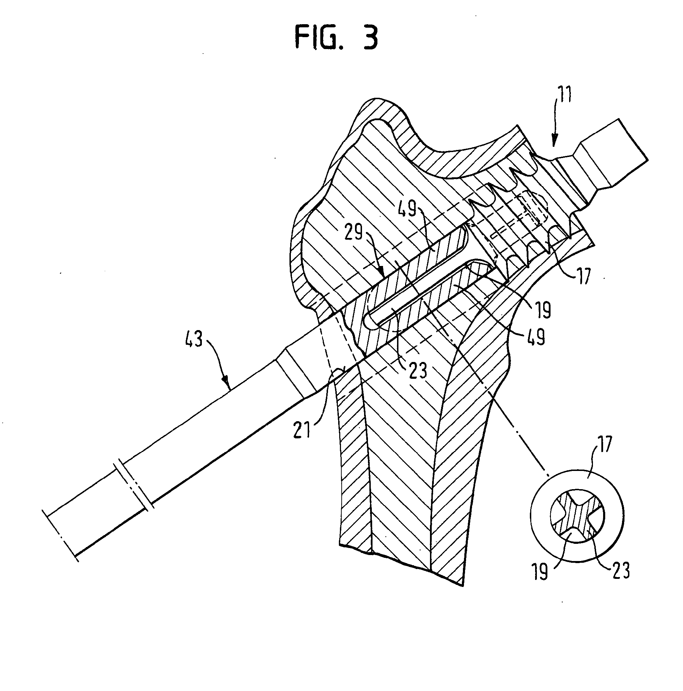

Gemäß Fig. 3 umfasst der erfindungsgemäße Instrumentensatz außerdem ein Eindrehinstrument 43, das auf den distalen Zementierabschnitt 29 des Prothesenschaftes 11 abgestimmt ist. Das Eindrehinstrument 43 ist mit Fingern 49 versehen, die in die Vertiefungen 19 des Zementierabschnitts 29 eingreifen. Das Eindrehinstrument 43 ist insofern komplementär zu dem Zementierabschnitt 29 des Schaftes 11 ausgebildet.

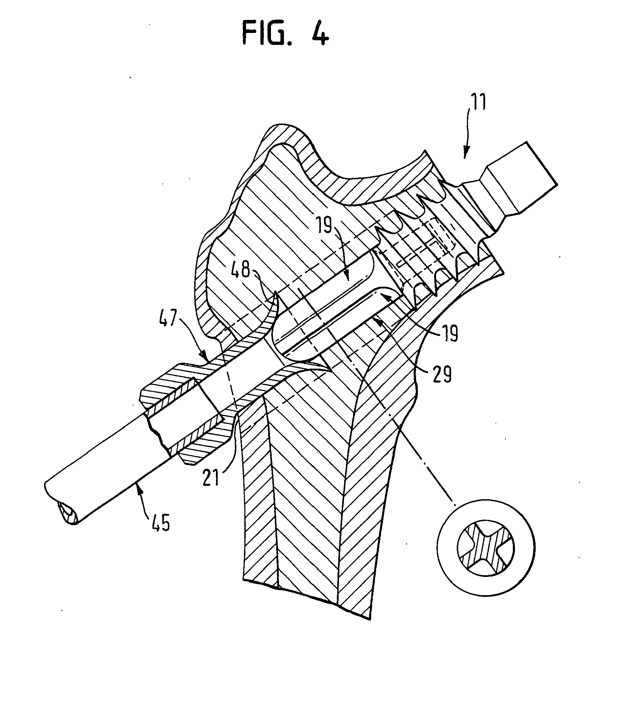

Zum Einzementieren des Prothesenschaftes 11 dient gemäß Fig. 4 ein Einbringinstrument 45 des erfindungsgemäßen Instrumentensatzes, das einen an die Formgebung des hinteren Endes des Zementierabschnitts 29 angepassten Einbringabschnitt 47 umfasst. Der Einbringabschnitt 47 ist als separates Bauteil vorgesehen, das auf eine herkömmliche Zementspritze oder -pistole aufgesteckt werden kann und somit als eine Art Adaptertülle dient.

Der Austrittsbereich des Einbringabschnitts 47 ist als glockenförmiger Kontaktbereich 48 ausgebildet, der radial über den lediglich den Kernlochdurchmesser aufweisenden Zementierabschnitt 29 vorsteht und durch seine Formgebung eine radiale Ausbreitung des eingespritzten Knochenzements unterstützt sowie ein Austreten von Knochenzement aus dem Durchgang 21 verhindert.

Der eingespritzte Knochenzement gelangt über den Einbringabschnitt 48 in die Vertiefungen 19 des Zementierabschnitts 29 und von dort in im Wesentlichen radialer Richtung in das offenporige Material der Spongiosa des Femurknochens.

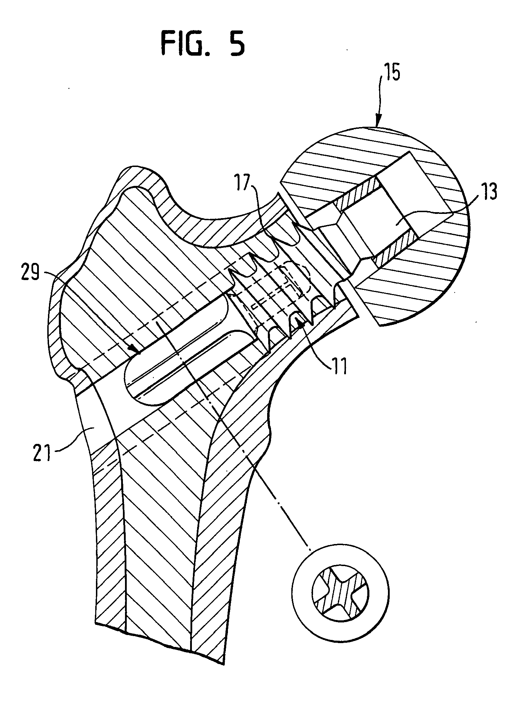

Im komplettierten Zustand gemäß Fig. 5 befindet sich der mit dem auf den Konus 13 aufgesetzten Prothesenkopf 15 versehene Prothesenschaft 11 in der korrekten Tiefe innerhalb des Durchgangs 21 und ist im relativ harten Bereich des Femurhalses über das proximale Gewinde 17 und in der relativ offenporigen Spongiosa über den Zementierabschnitt 29 am Femur verankert.

Erfindungsgemäß wird somit der Prothesenschaft 11 praktisch über seine gesamte axiale Länge im Femur fixiert, ohne dass externe Druck- oder Spanneinrichtungen erforderlich wären, um den Prothesenschaft 11 zu befestigen.

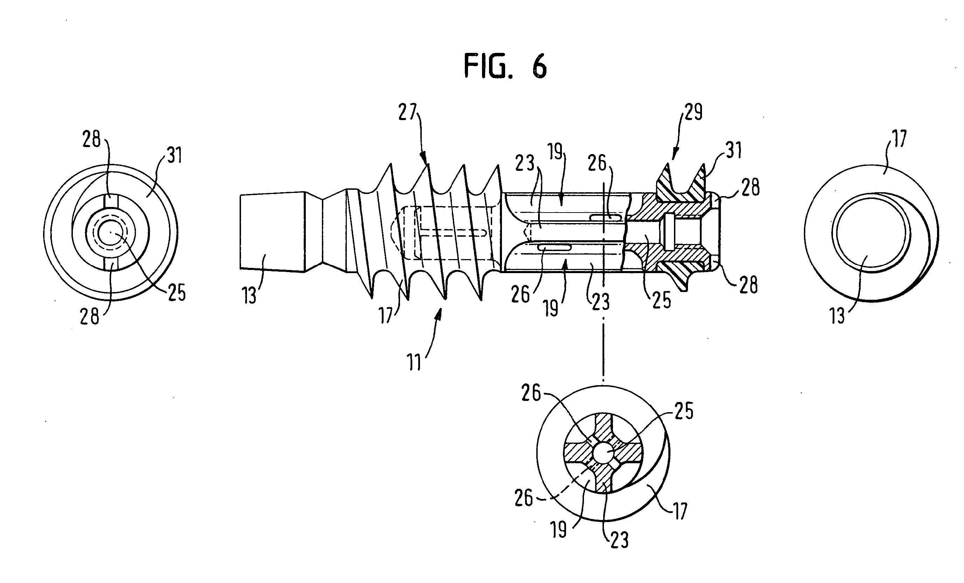

Die in Fig. 6 dargestellte zweite Ausführungsform eines Prothesenschaftes 11 unterscheidet sich von der zuvor beschriebenen ersten Ausführungsform durch die Ausgestaltung des Zementierabschnitts 29. Die wiederum vorgesehenen, sich in axialer Richtung zwischen stegartigen Wandabschnitten 23 erstreckenden Vertiefungen 19 sind hier nicht nach distal offen und werden nicht direkt mit Knochenzement beschickt. Vielmehr ist der Zementierabschnitt 29 mit einem axial verlaufenden, zentralen Einbringkanal 25 versehen, der von distal zugänglich ist und mit den Vertiefungen 19 über radiale Durchbrüche 26 kommuniziert. Diese Öffnungen 26 sind dabei bezüglich der axialen Richtung zueinander versetzt angeordnet.

Im Bereich seines distalen Endes ist der Zementierabschnitt 29 mit einem insbesondere durch Anspritzen hergestellten Gewindeabschnitt 31 versehen, der beim Einbringen des Knochenzements als Dichtung dient, worauf nachstehend näher eingegangen wird. Der Gewindeabschnitt 31 ist auf das proximale Verankerungsgewinde 17 abgestimmt und insbesondere als dessen Fortsetzung ausgelegt.

Da der primäre Zweck des Gewindeabschnitts 31 nicht die Verankerung des Schaftes 11 im Femurknochen ist, kann der Gewindeabschnitt 31 z.B. aus einem bioresorbierbaren Material hergestellt sein.

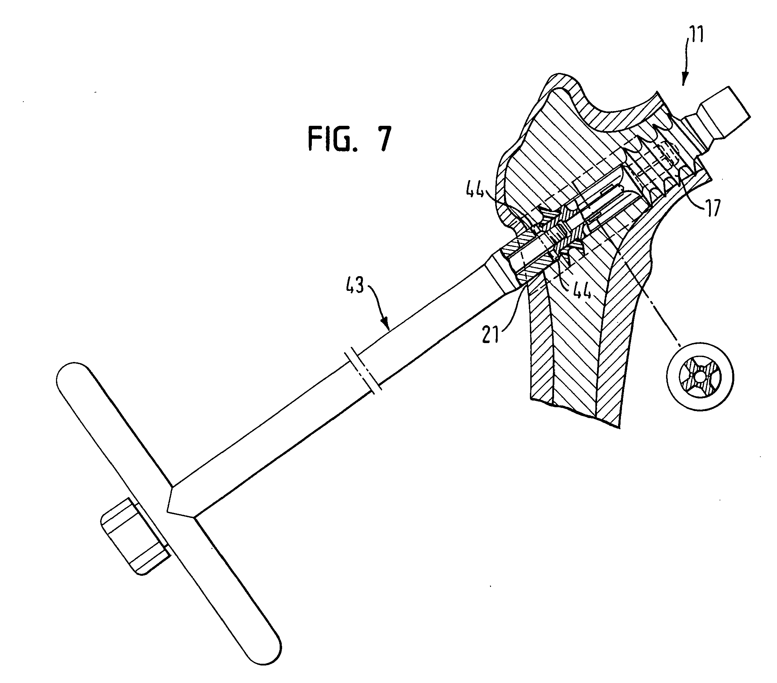

Die distale Stirnseite des Zementierabschnitts 29 ist mit zwei einander diametral gegenüberliegenden Aussparungen 28 versehen. Wie Fig. 7 zeigt, dienen diese Aussparungen 28 zur Aufnahme von entsprechend geformten Vorsprüngen 44 eines Eindrehinstruments 43, mit dem der Schaft 11 in den zuvor hergestellten Durchgang 21 im Femurknochen eingedreht wird.

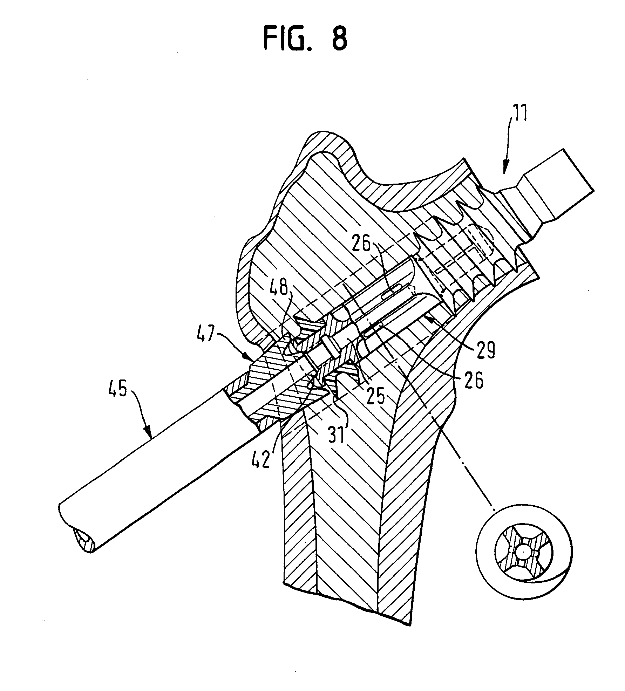

Nicht nur dieses Eindrehinstrument 43, sondern auch das Instrument 45 zum Einbringen von Knochenzement unterscheidet sich von dem entsprechenden Instrument für die erste Ausführungsform. Wie Fig. 8 zeigt, weist der das distale Ende des Zementierabschnitts 29 im aufgesetzten Zustand abdichtende Kontaktbereich 48 der Tülle 47 zusätzlich zu einer radial äußeren Ringwand, die das distale Ende des Zementierabschnitts 29 außen umfasst, eine radial innere Ringwand 42, die den Kanal 25 des Zementierabschnitts 29 nach außen abdichtet.

Anders als beim Einbringinstrument für die erste Ausführungsform des Prothesenschaftes hat bei dem in Fig. 8 dargestellten Einbringinstrument 45 die radial äußere Ringwand des Kontaktbereiches 48 keine Lenkungs- und Abdichtfunktion. Der axial verlaufende Kanal 25 und die in die Vertiefungen 19 mündenden Durchbrüche 26 sorgen für ein radiales Ausströmen des Knochenzementes aus den Vertiefungen 19. Die Abdichtung des Durchgangs 21 ist durch die als Gewinde ausgebildete Dichtung 31 gewährleistet.

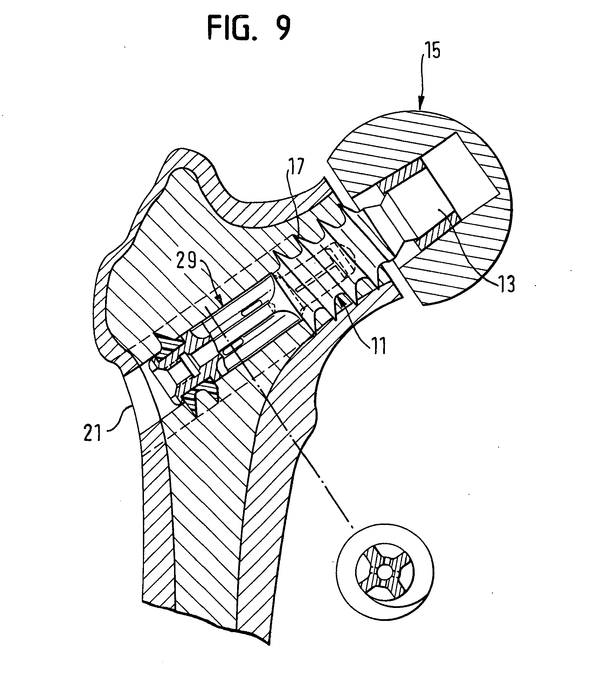

Somit ist auch der Prothesenschaft 11 der zweiten Ausführungsform gemäß Fig. 9 über das Gewinde 17 im vergleichsweise harten Material des Femurhalses und über den mit dem Zementierabschnitt 29 zusammenwirkenden Knochenzement im vergleichsweise offenporigen Material der Spongiosa am Femurknochen sicher verankert.

Unabhängig davon, welcher Zementierabschnitt 29 für den erfindungsgemäßen Prothesenschaft 11 zum Einsatz kommt, ist der Ablauf des Operationsverfahrens zum Einsetzen der erfindungsgemäßen Prothese wie folgt:

Zunächst wird über einen vorderen Zugang (anterior) der Femurkopf abgetrennt und herausgenommen. Anschließend wird entweder gleich das Acetabulum des Hüftknochens bearbeitet, und zwar ebenfalls durch den erwähnten vorderen Zugang, oder es wird zunächst die ohnehin benötigte Kernlochbohrung 51 (vgl. z.B. Fig. 2) ausgebildet. Dies erfolgt mit Hilfe eines computergestützten optischen Navigationssystems.

Zur Bearbeitung des Acetabulums über die Kernlochbohrung 51 wird in diese eine nicht dargestellte Bearbeitungshülse eingeschoben, über welche anschließend die für die Bearbeitung des Acetabulums benötigten Instrumente zugeführt werden können. Die hierbei erforderliche korrekte Ausrichtung des Femurknochens relativ zum Hüftknochen kann in vorteilhafter Weise dadurch fixiert werden, dass die Bearbeitungshülse derart bemessen wird, dass sie lateral aus dem Oberschenkel des Patienten herausragt und am Operationstisch durch geeignete Mittel festgehalten wird.

Das anschließende Einsetzen der nicht dargestellten Prothesenschale erfolgt wiederum über den erwähnten vorderen Zugang.

Dann wird nach Entnahme der Bearbeitungshülse mittels des Gewindeschneiders in die Kernlochbohrung 51 ein durchgehendes Innengewinde für den Prothesenschaft 11 ausgebildet. Anschließend wird mittels des Eindrehinstruments 43 (vgl. Fig. 3 bzw. Fig. 7) der Prothesenschaft 11 in eine zuvor im Rahmen der Operationsplanung festgelegte Tiefe eingedreht.

Daraufhin wird - wiederum über den erwähnten vorderen Zugang - der Prothesenkopf 15 eingesetzt und auf den Konus 15 des Prothesenschaftes 11 aufgesetzt. Auch die zur Fixierung des Kopfes 15 auf dem Konus 13 erforderlichen Schläge auf den Kopf 15 werden durch den vorderen Zugang mittels entsprechender, hier nicht dargestellter Instrumente vorgenommen.

Anschließend kann die korrekte Lage des Prothesenkopfes 15 kontrolliert und erforderlichenfalls durch Verdrehen des Prothesenschaftes 11 im Durchgang 21 korrigiert werden.

Ist die korrekte Lage, d.h. die korrekte Tiefe des Prothesenschaftes 11 im Durchgang 21, erreicht, wird der Prothesenschaft 11 mit Hilfe des Einbringinstruments 45 (vgl. Fig. 4 bzw. Fig. 8) einzementiert. Hierzu wird die jeweilige "Tülle" 47, die an einer herkömmlichen Zementspritze angebracht ist, auf das distale Ende des Prothesenschaftes 11 gesetzt, wie es vorstehend erläutert wurde. Sobald der Zement ausgehärtet ist, ist der Prothesenschaft 11 nicht nur über sein Gewinde 17, sondern außerdem über den Zementierabschnitt 29 sicher im Femurknochen verankert.

Ein wesentlicher Vorteil der Erfindung besteht darin, dass mit ausschließlich minimal invasiven Operationstechniken gearbeitet werden kann.

### Bezugszeichenliste

- 11: Schaft der Prothese
- 13: proximaler Endbereich, Kopplungsabschnitt, Konus
- 15: Kopf der Prothese
- 17: Gewinde des Prothesenschaftes
- 19: Vertiefung, Aufnahme
- 21: Durchgang
- 23: Wandabschnitt, Steg
- 25: Kanal
- 26: Öffnung, Durchbruch
- 27: Verankerungsabschnitt mit Gewinde
- 28: Aussparung für Eindrehinstrument
- 29: Verankerungsabschnitt mit Vertiefung, Zementierabschnitt
- 31: Dichtungsabschnitt, hinterer Gewindeabschnitt
- 33: Verbindungszapfen
- 35: Zapfenaufnahme
- 41: Gewindeschneider
- 42: radial innere Ringwand
- 43: Eindrehinstrument
- 44: Vorsprung des Eindrehinstruments
- 45: Einbringinstrument, Zementspritze bzw. -pistole
- 47: Einbringabschnitt, Tülle
- 48: Kontaktbereich
- 49: Finger des Eindrehinstruments
- 51: Kernlochbohrung

## Patentansprüche

1. Hüftgelenkprothese mit einem in den Femurhals einsetzbaren Prothesenschaft (11), der mit seinem proximalen, insbesondere konusförmig ausgebildeten Endbereich (13) mit einem Prothesenkopf (15) koppelbar ist, für den eine in den Hüftknochen einsetzbare Prothesenschale vorgesehen ist,
wobei der Schaft (11) an seinem Umfang wirksame Verankerungsmittel (17, 19) aufweist, über welche der Schaft (11) in einem durch den Femurhals verlaufenden Durchgang (21) verankerbar ist, in den der Schaft (11) von lateral her einbringbar ist.

2. Hüftgelenkprothese nach Anspruch 1,
**dadurch gekennzeichnet,**
**dass** der Schaft (11) an seinem Umfang mit einem Gewinde (17) versehen ist, über welches der Schaft (11) in den Durchgang (21) eindrehbar ist, wobei vorzugsweise die Längsposition des Schaftes (11) im Durchgang (21) über das Gewinde (17) einstellbar ist.

3. Hüftgelenkprothese nach Anspruch 1 oder 2,
**dadurch gekennzeichnet,**
**dass** der Schaft (11) an seinem Umfang mit wenigstens einer Vertiefung (19) zur Aufnahme von Knochenzement versehen ist.

4. Hüftgelenkprothese nach Anspruch 3,
**dadurch gekennzeichnet,**
**dass** die Vertiefung (19) zur Einbringung des Knochenzements über das distale Ende des Schaftes (11) zugänglich ist.

5. Hüftgelenkprothese nach Anspruch 3 oder 4,
**dadurch gekennzeichnet,**
**dass** die Vertiefung (19) eine lang gestreckte Form aufweist und parallel zur Schaftachse verläuft.

6. Hüftgelenkprothese nach einem der Ansprüche 3 bis 6,
**dadurch gekennzeichnet,**
**dass** eine Mehrzahl von in Umfangsrichtung verteilt angeordneten, jeweils durch einen insbesondere rippen- oder stegartigen Wandabschnitt (23) voneinander getrennten Vertiefungen (19) vorgesehen ist.

7. Hüftgelenkprothese nach einem der vorhergehenden Ansprüchen,
**dadurch gekennzeichnet,**
**dass** ein ein Gewinde (17) umfassender Verankerungsabschnitt (27) zwischen dem proximalen Kopplungsabschnitt (13) und einem distalen, eine Vertiefung (19) umfassenden Verankerungsabschnitt (29) gelegen ist.

8. Hüftgelenkprothese nach einem der vorhergehenden Ansprüche,
**dadurch gekennzeichnet,**
**dass** ein eine Vertiefung (19) umfassender Verankerungsabschnitt (29) sich bis zum distalen Ende des Schaftes (11) erstreckt.

9. Hüftgelenkprothese nach einem der vorhergehenden Ansprüche,
**dadurch gekennzeichnet,**
**dass** der Schaft (11) im Bereich eines eine Vertiefung (19) umfassenden Verankerungsabschnitts (29) mit einem von seinem distalen Ende ausgehenden und in die Vertiefung (19) mündenden Kanal (25) zum Einbringen des Knochenzements versehen ist.

10. Hüftgelenkprothese nach Anspruch 9,
**dadurch gekennzeichnet,**
**dass** für eine Mehrzahl von Vertiefungen (19) ein gemeinsamer Kanal (25) vorgesehen ist.

11. Hüftgelenkprothese nach einem der vorhergehenden Ansprüche,
**dadurch gekennzeichnet,**
**dass** der Schaft (11) an seinem distalen Ende mit einem Dichtungsabschnitt (31) versehen ist, der in radialer Richtung über einen eine Vertiefung (19) umfassenden Verankerungsabschnitt (29) hinausragt.

12. Hüftgelenkprothese nach Anspruch 11,
**dadurch gekennzeichnet,**
**dass** der Dichtungsabschnitt (31) als Gewinde ausgebildet ist.

13. Hüftgelenkprothese nach Anspruch 11 oder 12,
**dadurch gekennzeichnet,**
**dass** der Dichtungsabschnitt (31) aus einem bioresorbierbaren Material hergestellt ist.

14. Hüftgelenkprothese nach einem der vorhergehenden Ansprüche,
**dadurch gekennzeichnet,**
**dass** der Schaft (11) mehrteilig ausgebildet ist, wobei vorzugsweise ein ein Gewinde (17) umfassender Schaftteil (27) aus einem anderen Material hergestellt ist als ein eine Vertiefung (19) umfassender Schaftteil (29).

15. Instrumentensatz zum Einsetzen einer insbesondere die Merkmale eines der vorhergehenden Ansprüche aufweisenden Hüftgelenkprothese, die einen in den Femurhals einsetzbaren Prothesenschaft (11) umfasst, der mit seinem proximalen, insbesondere konusförmig ausgebildeten Endbereich (13) mit einem Prothesenkopf (15) koppelbar ist, für den eine in den Hüftknochen einsetzbare Prothesenschale vorgesehen ist, wobei der Schaft (11) an seinem Umfang wirksame Verankerungsmittel (17, 19) aufweist, über welche der Schaft (11) in einem durch den Femurhals verlaufenden Durchgang (21) verankerbar ist, in den der Schaft (11) von lateral her einbringbar ist, und
wobei der Instrumentensatz einen Kernlochbohrer und einen Gewindeschneider (41) zum Herstellen des Durchgangs (21) von lateral sowie ein Eindrehinstrument (43) zum Eindrehen des Schaftes (11) in den Durchgang (21) aufweist.

16. Instrumentensatz nach Anspruch 15,
**dadurch gekennzeichnet,**
**dass** ein Einbringinstrument (45) zum Einbringen von Knochenzement vorgesehen ist, das einen auf das distale Ende des Schaftes (11) aufsetzbaren Einbringabschnitt (47) aufweist.

17. Instrumentensatz nach Anspruch 16,
**dadurch gekennzeichnet,**
**dass** der Einbringabschnitt (47) derart auf das distale Ende des Schaftes (11) abgestimmt ist, dass im aufgesetzten Zustand ein im Schaft (11) ausgebildeter Kanal (25) gegenüber der Umgebung abgedichtet ist.

18. Instrumentensatz nach Anspruch 16 oder 17,
**dadurch gekennzeichnet,**
**dass** der Einbringabschnitt (47) zumindest bereichsweise kappen-, hauben- oder glockenförmig ausgebildet ist.

19. Instrumentensatz nach einem der Ansprüche 15 bis 17,
**dadurch gekennzeichnet,**
**dass** der Einbringabschnitt (47) ein separates, mit einer Zementspritze oder -pistole verbindbares Bauteil ist.
